# EUROPEAN PATENT APPLICATION

(11) **EP 3 903 889 A1**
(43) Date of publication of application: **03.11.2021**
(21) Application number: 21167777.8
(22) Date of filing: 12.04.2021
(51) Int. Cl.: A62B 18/00, A62B 18/02, A62B 18/04, A41D 13/11, A61L 2/10

(54) **AIR MASK**

(30) Priority: 29.04.2020 US 202063017294 P
(71) Applicant: E2E, Mfg. LLC, Fremont CA 94538 (US)
(72) Inventor: Fajardo, Iggoni, CA, 94538 (US)
(74) Representative: Wang, Bo

(57) **Abstract**

An air mask is applied for a user to wear and includes a wearable main body, an airflow generator and a face shield. The wearable main body has a front part and a rear part and includes an airflow channel, an air inputting port disposed on the rear part, and an air outputting port disposed on the front part. The airflow generator is coupled to the air inputting port and configured for generating and guiding airflow to the airflow channel. The face shield is coupled to the front part of the wearable main body and has an air chamber communicated with the air outputting port. When the user wears the air mask, the air inputting port is located on rear head of the user, the air outputting port is located on forehead of the user, and the airflow flows downward from the forehead of the user to the air chamber.

## Description

### BACKGROUND OF THE INVENTION

### 1. Field of the invention

The present invention relates to a mask, and more particularly, to an air mask that can block the viruses and various suspended particles.

### 2. Description of the prior art

Air is one of the important elements to people for living. However, the air pollution is getting worse caused by industries development. Therefore, there are many dusts, and various suspended particles in the air which may be harmful to the human body. In addition, the air is also considered as a media for spreading harmful substance and viruses, such as tuberculosis, SARS, MERS and COVID-19.

In order to prevent droplet infection, medical staffs and normal persons often wear masks to protect themselves from inhalation of viruses or pollutants in the air. However, most medical masks are disposable masks which cannot be reused, resulting in the waste of resources. Moreover, most the medical masks directly cover the nose and mouth of the user, thereby decreasing the comfortabiliy. Although the medical masks with air permeability, the cavity between the mask and the user's face is still small. Therefore, the user may re-inhale the exhaust air, thereby decreasing practicality and comfortability. In addition, the medical masks do not completely cover the user's nose and mouth, so that the user may still inhale the unblocked harmful suspended particles and viruses. Therefore, it is necessary to develop a new type of mask to solve the problems.

### SUMMARY OF THE INVENTION

Therefore, the present invention provides an air mask to solve the problems of the prior art.

In one embodiment of the present invention, the air mask is applied for a user to wear and includes a wearable main body, an airflow generator and a face shield. The wearable main body is configured to be worn by the user. The wearable main body has a front part and a rear part and includes an airflow channel, an air inputting port and an air outputting port. The air inputting port is disposed on the rear part and communicated with an external environment, and the air outputting port is disposed on the front part. The airflow generator is coupled to the air inputting port and configured for generating and guiding airflow to the airflow channel. The face shield is coupled to the front part of the wearable main body. The face shield has an air chamber communicated with the air outputting port. When the user wears the wearable main body, the air inputting port is located on rear head of the user, the air outputting port is located on forehead of the user, the face shield is located in front of face of the user, and the airflow flows downward from the forehead of the user to the air chamber.

Wherein, the air mask further includes a filter coupled to the airflow channel and configured to filter air in the airflow channel.

Wherein, the air mask further includes an ultraviolet light generator coupled to the airflow channel. The ultraviolet light generator is configured to emit an ultraviolet light into the airflow channel.

Wherein, the wearable main body is a helmet.

Furthermore, the wearable main body has an inner surface and includes a first fixing structure and a second fixing structure configured on the inner surface. When the user wears the wearable main body, the first fixing structure and the second fixing structure lean against on the rear head and the forehead of the user respectively.

Wherein, the face shield includes a transparent portion, a side structure and a bottom structure. When the user wears the wearable main body, the transparent portion, the side structure, and the bottom structure form the air chamber in front of the face of the user.

Wherein, the material of the bottom structure is fabric material with air permeability, and the airflow flows from the air chamber to the external environment through the bottom structure.

Wherein, the transparent portion includes ventilation hole, and the airflow flows from the air chamber to the external environment through the ventilation hole.

Wherein, the air mask further includes a controller coupled to the airflow generator. The controller is configured to control the airflow generator to generate the airflow.

Furthermore, the controller is a portable device. The controller is wireless connected to the airflow generator to remotely control the airflow generator.

In summary, the air mask of the present invention can increase the practicality and filter efficiency, and protect the health of users through the filter and the ultraviolet light generator. Moreover, the air mask of the present invention can block the unfiltered air to enter the face shield with the enclosed design, thereby increasing the filter quality and efficiency. Furthermore, the air mask of the present invention can increase the convenience and comfortability through the elastic structures and the flexible fixing structures of the wearable main body and face shield. Moreover, the air mask of the present invention also can increase the filter quality and efficiency through the downward direction airflow and ventilation holes and bottom structure. In addition, the air mask of the present invention can increase convenience through the control mechanism.

### BRIEF DESCRIPTION OF THE APPENDED DRAWINGS

FIG. 1 is a schematic diagram illustrating an air mask according to an embodiment of the present invention.
FIG. 2 is a schematic diagram illustrating the side view of the air mask in FIG. 1.
FIG. 3 is a sectional schematic diagram illustrating the air mask in FIG. 1.
FIG. 4 is a schematic diagram illustrating the user wears the air mask of the present invention.
FIG. 5 is a schematic diagram illustrating the wearable main body of the air mask in FIG. 1.
FIG. 6 is a schematic diagram illustrating the air mask and a controller according to an embodiment of the present invention.
FIG. 7 is a schematic diagram illustrating the air mask and the controller according to another embodiment of the present invention.
FIG. 8 is a schematic diagram illustrating an air mask according to another embodiment of the present invention.

### DETAILED DESCRIPTION OF THE INVENTION

For the sake of the advantages, spirits and features of the present invention can be understood more easily and clearly, the detailed descriptions and discussions will be made later by way of the embodiments and with reference of the diagrams. It is worth noting that these embodiments are merely representative embodiments of the present invention, wherein the specific methods, devices, conditions, materials and the like are not limited to the embodiments of the present invention or corresponding embodiments. Moreover, the devices in the figures are only used to express their corresponding positions and are not drawing according to their actual proportion.

In the description of the present invention, it is to be understood that the orientations or positional relationships of the terms "longitudinal, lateral, upper, lower, front, rear, left, right, top, bottom, inner, outer" and the like are based on the orientation or positional relationship shown in the drawings. It is merely for the convenience of the description of the present invention and the description of the present invention, and is not intended to indicate or imply that the device or component referred to has a specific orientation, is constructed and operated in a specific orientation, and therefore cannot be understood as limitations of the invention.

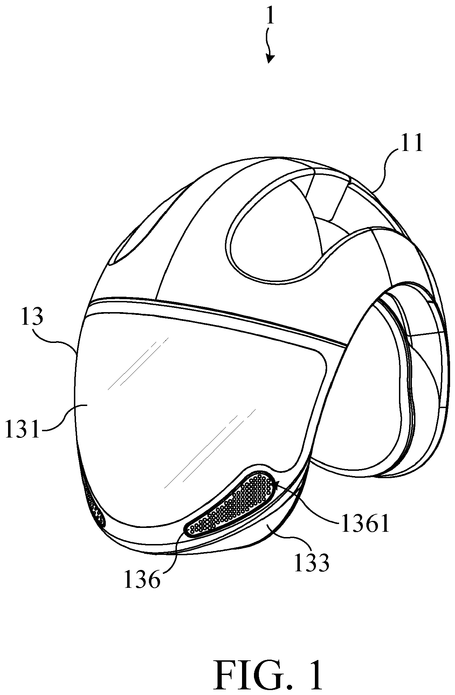

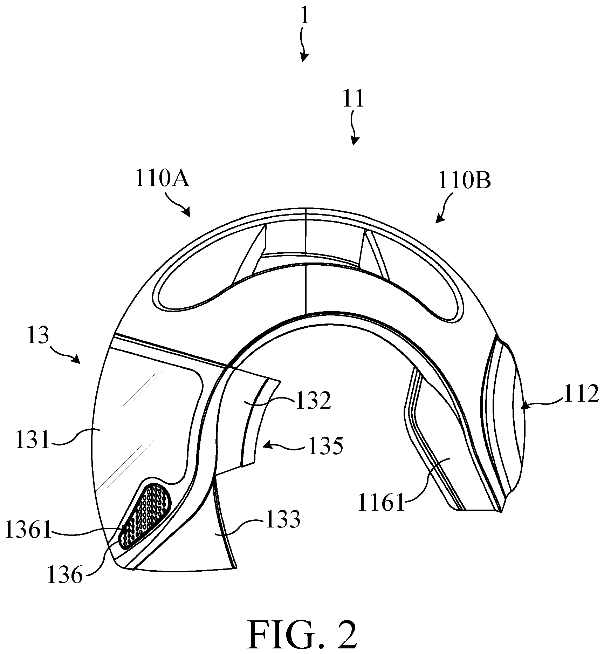

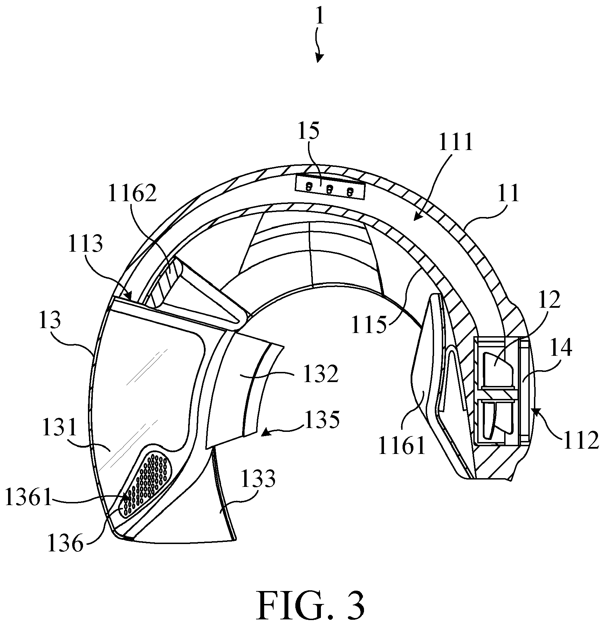

Please refer to FIG. 1, FIG. 2 and FIG. 3. FIG. 1 is a schematic diagram illustrating an air mask 1 according to an embodiment of the present invention. FIG. 2 is a schematic diagram illustrating the side view of the air mask 1 in FIG. 1. FIG. 3 is a sectional schematic diagram illustrating the air mask 1 in FIG. 1. As shown in FIG. 1, FIG. 2 and FIG. 3, the air mask 1 includes a wearable main body 11 and a face shield 13. The wearable main body 11 is configured to be worn by a user and has a front part 110A and a rear part 110B. Furthermore, the wearable main body 11 includes an airflow channel 111, an air inputting port 112 and an air outputting port 113. The air inputting port 112 and the air outputting port 113 are connected to two ends of the airflow channel 111 respectively. The air inputting port 112 is disposed on the rear part 110B and communicated with an external environment, and the air outputting port 113 is disposed on the front part 110A. The face shield 13 is coupled to the front part 110A of the wearable main body 11. The face shield 13 has an air chamber 135, and the air chamber 135 is communicated with the air outputting port 113.

In practice, the wearable main body 11 is a helmet. The user can wear the wearable main body 11 on the user's head, and the airflow channel 111 may be located above of the user's head. As shown in FIG. 3, the airflow channel 111 can be configured on the front part 110A and the rear part 110B of the wearable main body 11 to connect the air inputting port 112 and the air outputting port 113. That is to say, the airflow channel 111 can be configured along the shape of the helmet. The face shield 13 can be extended from the front part 110A of the wearable main body 11. Furthermore, the face shield 13 can be integrally formed on the wearable main body 11, but it is not limited thereto. Because the air inputting port 112 is communicated with the external environment and the air outputting port 113 is communicated with the air chamber 135, the air chamber 135 can be communicated with the external environment via the airflow channel 111. That is to say, the air from the external environment can pass through the airflow channel 111 to the air chamber 135.

In this embodiment, the air mask 1 further includes an airflow generator 12 coupled to the air inputting port 112. The airflow generator 12 is configured for generating and guiding the airflow to the airflow channel 111. In practice, the airflow generator 12 may be a fan. The airflow generator 12 can guide the air from the external environment into air inputting port 112. As shown in FIG. 3, the airflow generator 12 is disposed in the airflow channel 111 and close to the air inputting port 112, but it is not limited hereto. The airflow generator 12 also can be disposed in any position of the airflow channel 111. When the airflow generator 12 guides the air from the external environment into air inputting port 112, the airflow generator 12 also generates the airflow passing through the airflow channel 111, then the airflow passes through the air outputting port 113 and to the air chamber 135.

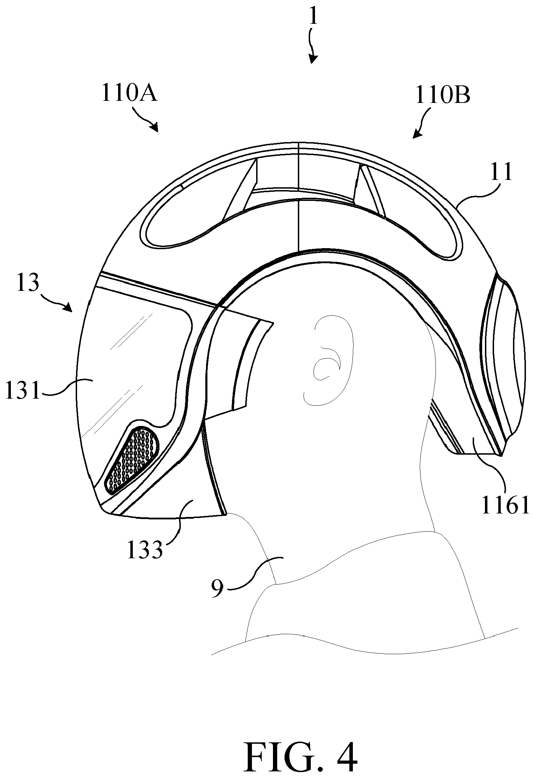

Please refer to FIG. 3 and FIG. 4. FIG. 4 is a schematic diagram illustrating the user 9 wears the air mask 1 of the present invention. As shown in FIG. 3 and FIG. 4, when the user 9 wears the air mask 1, the front part 110A of the wearable main body 11 is located on the front head of the user 9, the rear part 110B of the wearable main body 11 is located on the rear head of the user 9, and the face shield 13 is located in front of the user's face. Furthermore, the air inputting port 112 is located on the rear head of the user 9, and the air outputting port 113 is located on the front head of the user 9.

In practice, the air inputting port 112 may be located right behind the user's head (as shown in FIG. 4), but it is not limited thereto. The position of the air inputting port 112 also may be located on any position of the rear head of the user 9. Moreover, the air outputting port 113 may be located on the forehead of the user 9, and the direction of the air outputting port 113 is facing the ground. Therefore, when the airflow generator 12 guides the air from the external environment into air inputting port 112 and generates the airflow in the airflow channel 111, the airflow may flow downward from the airflow channel 111 to the air chamber 135.

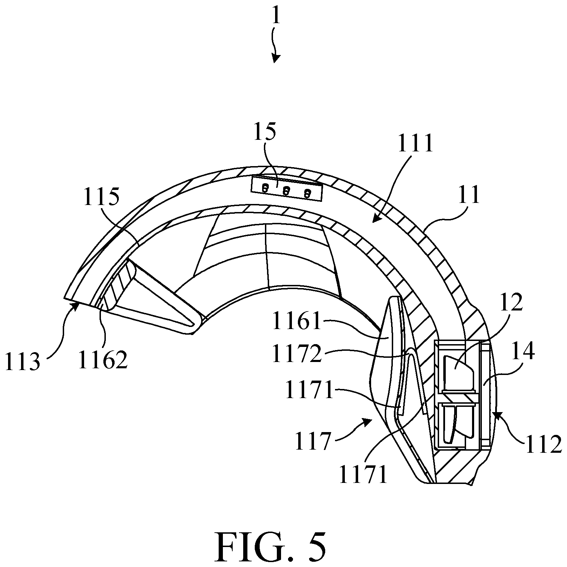

Please refer to FIG. 3, FIG. 4 and FIG. 5. FIG. 5 is a schematic diagram illustrating the wearable main body 11 of the air mask 1 in FIG. 1. As shown in FIG. 3, FIG. 4 and FIG. 5, in this embodiment, the wearable main body 11 has an inner surface 115 and includes a first fixing structure 1161 and a second fixing structure 1162. The first fixing structure 1161 and the second fixing structure 1162 are configured on the inner surface 115. Furthermore, the first fixing structure 1161 is configured on the rear part 110B, and the second fixing structure 1162 is configured on the front part 110A.

In practice, the first fixing structure 1161 and the second fixing structure 1162 may be protruded from the inner surface 115 or installed on the inner surface 115. The first fixing structure 1161 and the second fixing structure 1162 may be formed by plastic materials such as EVA foam (Ethylene-Vinyl Acetate foam) and PVC foam (Polyvinyl chloride foam). Furthermore, the sizes of the first fixing structure 1161 and the second fixing structure 1162 can be corresponding to the size of the user's head. As shown in FIG. 4, the first fixing structure 1161 is located on the rear part 110B corresponding to the rear head of the user 9, and the second fixing structure 1162 is located on the front part 110A corresponding to the forehead of the user 9. When the user 9 wears the air mask 1, the inner surface 115 of the wearable main body 11 is facing to the user's head, and the first fixing structure 1161 and the second fixing structure 1162 contact and lean against on the rear head and the forehead of the user 9 respectively.

In this embodiment, as shown in FIG. 5, the wearable main body 11 further includes an elastic structure 117. The elastic structure 117 is configured between the inner surface 115 and the first fixing structure 1161. Furthermore, the elastic structure 117 includes a first arm 1171A, a second arm 1171B, and a bending portion 1172 connected to the first arm 1171A and the second arm 1171B. The first arm 1171A is connected to the inner surface 115 and the second arm 1171B is connected to the first fixing structure 1161. In practice, when the user wears the air mask 1, the rear head of the user contacts and pushes the first fixing structure 1161 toward the inner surface 115 direction. At this time, the elastic structure 117 generates an elastic force when the elastic structure 117 is compressed by the first fixing structure 1161. Then, the elastic structure 117 pushes back the first fixing structure 1161 according to the elastic force to contact and lean against the rear head of the user, to fix the wearable main body 11 on the user' head. It should be noted that the type of the elastic structure is not limited in this embodiment, the elastic structure also can be a spring. Therefore, the air mask of the present invention can increase the convenience and comfort through the elastic structure and the flexible fixing structures.

In this embodiment, the air mask 1 further includes a filter 14 coupled to the airflow channel 111 and configured to filter air in the airflow channel 111. In practice, the filter 14 may be a HEPA filter (high efficiency particulate air filter), an activated carbon filter or any filter with air cleaning function. When the air passes through the filter 14, the filter 14 can filter suspended particles (such as PM2.5 particles, bacteria, pollen and other invisible allergens) in the air. As shown in FIG. 3, the filter 14 is disposed in the airflow channel 111 and between the airflow generator 12 and the air inputting port 112. When the airflow generator 12 guides air from the external environment to the air inputting port 112, the filter 14 will filter the air first, and the filtered air flows into the airflow channel 111, then the filtered air flows to the air chamber 135, so that the user can inhale clean air. Therefore, the air mask of the present invention can increase the practicality and filter efficiency, and protect the health of users. It should be noted that the filter not only can be disposed between the airflow generator and the air inputting port, the filter also can be disposed in any position of the airflow channel.

In this embodiment, the air mask 1 further includes an ultraviolet light generator 15 coupled to the airflow channel 111. The ultraviolet light generator 15 is configured to emit an ultraviolet light into the airflow channel 111. In practice, when the airflow generator 12 guides air from the external environment to the airflow channel 111, the air passes through the ultraviolet light emitted by the ultraviolet light generator 15. At this time, the ultraviolet light can kill the virus and bacteria in the air to generate the clean air. Then, the clean air flows to the air chamber 135, and the user can inhale clean air. Therefore, the air mask of the present invention can increase the practicality and filter efficiency, and protect the health of users. It should be noted that the ultraviolet light generator not only can be disposed on the position in FIG. 3, the ultraviolet light generator also can be disposed in any position of the airflow channel.

Please refer from FIG. 1 to FIG. 4. In this embodiment, the face shield 13 includes a transparent portion 131, a side structure 132 and a bottom structure 133. Furthermore, the transparent portion 131, the side structure 132 and the bottom structure 133 form the air chamber 135. When the user 9 wears the face shield 13, the transparent portion 131 is located in front of the face of the user 9, the side structure 132 may contact the cheek of the user 9, and the bottom structure 133 may contact the chin of the user 9.

In practice, the side structure 132 may be formed by plastic materials such as EVA foam (Ethylene-Vinyl Acetate foam) and PVC foam (Polyvinyl chloride foam). When the user 9 wears the air mask 1, the side structure 132 will deform according to the face shape of the user, and only contact and attach on the face of the user 9 instead of forcibly resisting, thereby increasing the convenience and comfortability. Furthermore, the material of the bottom structure 133 can be fabric material with permeability such as clothes, and the bottom structure 133 also can optionally cover the chin of the user 9. Therefore, the air mask 1 of the present invention can block the unfiltered air to enter the air chamber 135, and the user 9 can inhale the clean air, thereby increasing filter efficiency and filter quality.

In practice, when the user 9 generates carbon dioxide air or exhaust air during breathing, the carbon dioxide air or exhaust air may pass through the bottom structure 133 to the external environment. Because the clean air outputted from the air outputting port 112 flows downward to the user's face, and the user's face is located between the air outputting port 112 and the bottom structure 133, the user 9 can inhale the clean air before the clean air passes through the bottom structure 133 to the external environment. At this time, part of the clean air will also push the carbon dioxide air or exhaust air generated by the user 9 to pass through the bottom structure 133. Furthermore, because the airflow only flows in the one downward direction, the air chamber does not generate the turbulence. That is to say, the user will not re-inhale the carbon dioxide air or exhaust air. Therefore, the air mask of the present invention can increase the filter quality and efficiency.

Moreover, in this embodiment, the transparent portion 131 of the face shield 13 further includes ventilation holes 1361. The airflow flows from the air chamber 135 to the external environment through the ventilation holes 1361. In practice, as shown in FIG. 1, the transparent portion 131 may include two ventilation structures 136, and each ventilation structure 136 includes a plurality of ventilation holes 1361. Furthermore, the ventilation structures 136 can be located on the lower position of the transparent portion 131, and the user's face is located between the air outputting port 112 and the ventilation structure 136. Therefore, the user 9 can inhale the clean air before the clean air passes through the ventilation holes 1361 to the external environment. Similarly, because the airflow only flows in the one downward direction, the user will not re-inhale the carbon dioxide air or exhaust air, and part of the clean air also will push the carbon dioxide air or exhaust air to pass through the ventilation holes 1361. Therefore, the air mask of the present invention can increase the filter quality and efficiency. It should be noted that the shape and number of the ventilation structures are not limited in FIG. 1.

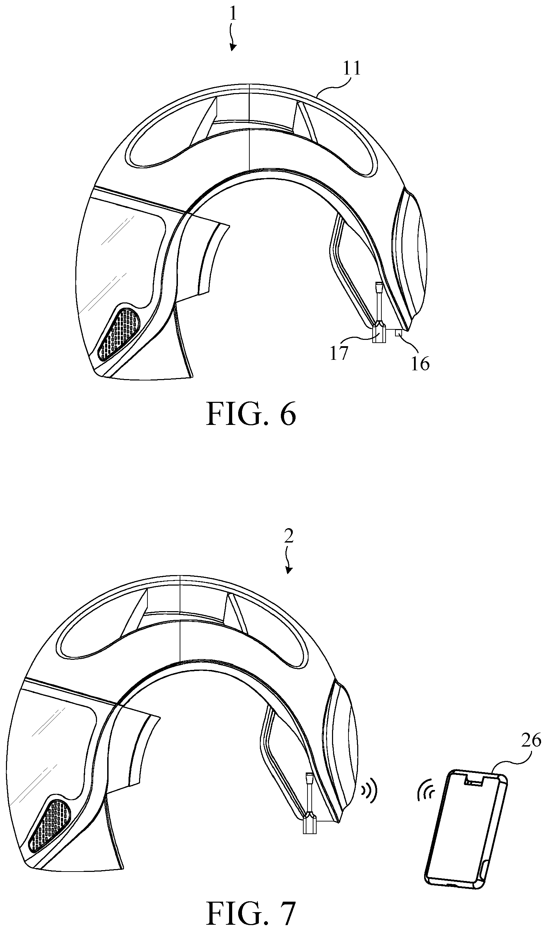

In addition to the above-mentioned components, the air mask of the present invention may also include a control mechanism. Please refer to FIG. 6. FIG. 6 is a schematic diagram illustrating the air mask 1 and a controller 16 according to an embodiment of the present invention. In this embodiment, the air mask 1 further includes a controller 16 coupled to the airflow generator and disposed on the bottom side of the wearable main body 11. The controller 16 is configured to control the airflow generator to generate the airflow. In practice, the controller 16 may be a switch, a push button, a touch switch, a knob or any component with on /off function. Therefore, the user can control the airflow generator by pressing or touching the controller 16. Furthermore, the controller 16 also can be coupled to the ultraviolet light generator and control the ultraviolet light generator to generate the ultraviolet light. In one embodiment, the airflow generator is a variable speed fan, and the controller can control the speed of the airflow. It is should be noted that the position of the controller 16 is not limited hereto, the controller 16 may be disposed on any position of the wearable main body 11.

Moreover, the air mask 1 further includes a circuit board and a power supply module (not shown in figure). In this embodiment, the circuit board is disposed in the wearable main body, and the circuit board is connected to the airflow generator, the ultraviolet light generator, the controller 16 and the power supply module. Furthermore, the power supply module includes a battery and a rechargeable component 17 configured for providing electrical power to the airflow generator and the ultraviolet light generator. In practice, the battery may be a secondary battery or rechargeable battery, and the rechargeable component 17 may be a recharging cable. The recharging cable may receive and transmit the electrical power to the battery. Therefore, the controller 16 can control the airflow generator and the ultraviolet light generator through the circuit board, and the power supply module can drive the airflow generator and the ultraviolet light generator through the circuit board.

The type of the controller is not limited to the aforementioned embodiment, the controller also can be another type. Please refer to FIG. 7. FIG. 7 is a schematic diagram illustrating the air mask 2 and the controller 26 according to another embodiment of the present invention. The difference between this embodiment and the aforementioned embodiment is that the controller 26 is a portable device and the air mask 2 includes a transceiver (not shown in figure). Furthermore, the transceiver is connected to the airflow generator. The controller 26 is wireless connected to the transceiver to remotely control the airflow generator. In practice, the controller 26 may be a mobile phone or a tablet, and the controller 26 and the transceiver can be connected in Wi-Fi, Zigbee, Bluetooth or NFC. Therefore, the user can control the airflow generator by the mobile phone or the tablet. Furthermore, the transceiver also can be connected to the ultraviolet light generator, so that the user can control the ultraviolet light generator to generate the ultraviolet light through the portable device. Therefore, the air mask of the present invention can increase convenience through the control mechanism.

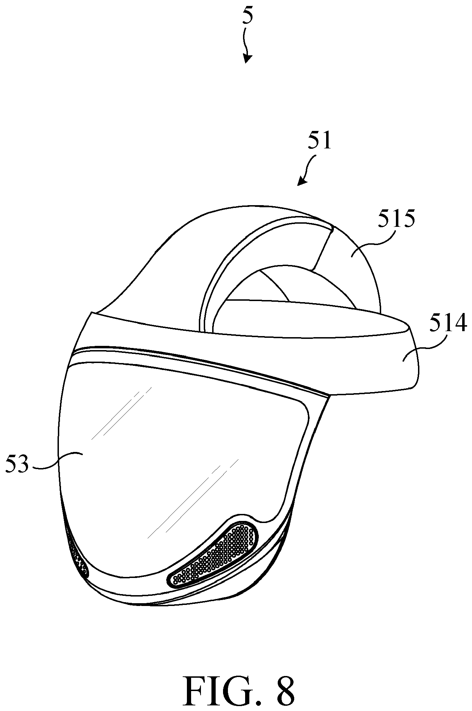

In addition to the aforementioned structure, the air mask also can be another structure. Please refer to FIG. 8. FIG. 8 is a schematic diagram illustrating an air mask 5 according to another embodiment of the present invention. The difference between this embodiment and the aforementioned embodiment is that the wearable main body 51 includes a circular telescopic belt 514 and an airflow structure 515, and the airflow structure 515 is configured above the circular telescopic belt 514. In practice, the airflow channel is configured in the airflow structure 515, and the face shield 53 is disposed on one side of the circular telescopic belt 514 and connected to the airflow structure 515. The airflow generator is disposed on the other side of the circular telescopic belt and opposite to the face shield 53. When the user wears the air mask 5, the circular telescopic belt 514 of the wearable main body 51 is arranged on the user's head, the face shield 53 is located in front of the face of the user, and the airflow generator is located on rear head of the user. The functions of the airflow generator and the face shield are the same with the functions of the corresponding components in the aforementioned embodiment, it will not be scribed herein.

In summary, the air mask of the present invention can increase the practicality and filter efficiency, and protect the health of users through filter and the ultraviolet light generator. Moreover, the air mask of the present invention can block the unfiltered air to enter the face shield with the enclosed design, thereby increasing the filter quality and efficiency. Furthermore, the air mask of the present invention can increase the convenience and comfortability through the elastic structures and the flexible fixing structures of the wearable main body and face shield. Moreover, the air mask of the present invention also can increase the filter quality and efficiency through the downward direction airflow and ventilation holes and bottom structure. In addition, the air mask of the present invention can increase convenience through the control mechanism.

With the examples and explanations mentioned above, the features and spirits of the invention are hopefully well described. More importantly, the present invention is not limited to the embodiment described herein. Those skilled in the art will readily observe that numerous modifications and alterations of the device may be made while retaining the teachings of the invention. Accordingly, the above disclosure should be construed as limited only by the metes and bounds of the appended claims.

## Claims

1. An air mask, applied for a user to wear, the air mask comprising:
a wearable main body, configured to be worn by the user, the wearable main body having a front part and a rear part and comprising an airflow channel, an air inputting port and an air outputting port, the air inputting port being configured on the rear part and communicated with an external environment, and the air outputting port being configured on the front part;
an airflow generator, coupled to the air inputting port, the airflow generator being configured for generating and guiding airflow to the airflow channel; and
a face shield, coupled to the front part of the wearable main body, the face shield having an air chamber, and the air chamber being communicated with the air outputting port;
wherein, when the user wears the wearable main body, the air inputting port is located on rear head of the user, the air outputting port is located on forehead of the user, the face shield is located in front of face of the user, and the airflow flows downward from the forehead of the user to the air chamber.

2. The air mask of claim 1, further comprising a filter coupled to the airflow channel and configured to filter air in the airflow channel.

3. The air mask of claim 1, further comprising an ultraviolet light generator coupled to the airflow channel, the ultraviolet light generator being configured to emit an ultraviolet light into the airflow channel.

4. The air mask of claim 1, wherein the wearable main body is a helmet.

5. The air mask of claim 4, wherein the wearable main body has an inner surface and comprises a first fixing structure and a second fixing structure configured on the inner surface, when the user wears the wearable main body, the first fixing structure and the second fixing structure lean against on the rear head and the forehead of the user respectively.

6. The air mask of claim 1, wherein the face shield comprises a transparent portion, a side structure and a bottom structure, when the user wears the wearable main body, the transparent portion, the side structure, and the bottom structure form the air chamber in front of the face of the user.

7. The air mask of claim 6, wherein the material of the bottom structure is fabric material with air permeability, and the airflow flows from the air chamber to the external environment through the bottom structure.

8. The air mask of claim 6, wherein the transparent portion comprises a ventilation hole, and the airflow flows from the air chamber to the external environment through the ventilation hole.

9. The air mask of claim 1, further comprising a controller coupled to the airflow generator, the controller being configured to control the airflow generator to generate the airflow.

10. The air mask of claim 9, wherein the controller is a portable device, and the controller is wireless connected to the airflow generator to remotely control the airflow generator.
